(19) Europäisches Patentamt
European Patent Office
Office européen des brevets

(11) Publication number: 0 184 361 B1

# (12) EUROPEAN PATENT SPECIFICATION

(45) Date of publication of patent specification: 02.01.91

(51) Int. Cl.$^5$: C 08 B 37/12, C 07 K 17/06, C 12 N 11/10, C 07 K 3/20

(21) Application number: 85308501.7

(22) Date of filing: 22.11.85

## (54) Method of activating polymeric carrier.

(30) Priority: 07.12.84 US 679167
07.12.84 US 679525

(43) Date of publication of application:
11.06.86 Bulletin 86/24

(45) Publication of the grant of the patent:
02.01.91 Bulletin 91/01

(84) Designated Contracting States:
AT BE CH DE FR GB IT LI LU NL SE

(56) References cited:
US-A-4 048 416
CHROMATOGRAPHIA, vol. 13, no. 5, May 1980, pages 295-297, Friedr. Vieweg & Sohn Verlagsgesellschaft mbH; E. BALD et al.: "Analytical utility of 2-halopyridinium salts, part III; paper electrophoretic characterization of alcohols as 2-alkoxy-1-methylpyridinium p-toluenesulphonates"

(73) Proprietor: BIOPROBE INTERNATIONAL, INC.
2842 Walnut Avenue Suite C
Tustin California 92680 (US)

(72) Inventor: Ngo, That T.
15 Deer Creek
Irvine California 92714 (US)

(74) Representative: Newstead, Michael John et al
Page & Co. Temple Gate House Temple Gate
Bristol BS1 6PL (GB)

(56) References cited:
CHEMICAL ABSTRACTS, vol. 86, 1977, page 467, abstract no. 138863r, Columbus, Ohio, US; K. HOJO et al.: "New synthetic reactions based on 1-methyl-2-fluoropyridinium salts. Facile conversion of alcohols to thioalcohols", & CHEM. LETT. 1977, (2), 133-6

CHEMICAL ABSTRACTS, vol. 90, 1979, page 635, abstract no. 15810w, Columbus, Ohio, US; A. MIYAGAWA et al.: "Preparation of various types of "activated thiol gel" and some properties for covalent chromatography", & BUNSEKI KAGAKU 1978, 27(5), 302-9

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

Field of the invention

This invention relates to a method of covalently binding organic ligands to polymeric carriers. In one of its more particular aspects the invention relates to a new method of covalently binding organic ligands containing one or more primary or secondary amino or sulfhydryl groups to polymeric gels.

Background and summary of the invention

The need for purifying various biologically active materials in a facile manner has long been appreciated. Early methods of enzyme purification, for example, were cumbersome and time consuming. Recently it has been found that enzymes and other biologically active materials can be purified by a process which involves immobilization of the enzyme or other biologically active material which will be referred to herein as a ligand, followed by separation of the immobilized ligand from the mixture in which it is present. The ligand can then be used in its immobilized form, if desired, or can be released from the carrier on which it is immobilized by suitable chemical treatment and used in its non-immobilized form. The discovery of methods for covalently bonding ligands to polymeric carriers has advanced the practice of enzymology, immunology, and various other biological techniques.

One of the first methods for immobilizing biological ligands involved treatment of a polymer containing hydroxyl groups with an activating agent such as cyanogen bromide, CNBr. The activated polymer could then be used to directly bind various biological ligands to the polymer by means of covalent bonds. Porath et al. describe several chemical activation methods including the CNBr method in Porath, et al., "Immobilized Enzymes. Methods in Enzymology, K. Mosbach, Ed., Vol. 44, p. 19—45, Academic Press, New York (1976). Most of the early methods for activating polymers containing hydroxyl groups were subject to certain disadvantages which made their widespread use impractical. In particular CNBr activation procedures suffer from the following disadvantages: (1) the linkages formed between CNBr-activated hydroxyl containing polymers and the amino groups of ligands which are reacted with the activated polymers are labile; (2) the reaction between the activated polymer and ligand frequently results in the introduction of charged species which interfere with utilization of the reaction product in affinity absorption; (3) CNBr is a noxious, lachrimatory and poisonous chemical which requires special care in its handling.

Efforts to find another method other than the CNBr method for coupling ligands to hydroxyl containing polymers resulted in the use of a number of different reagents including triazine trichloride, N-hydroxy succinimide 1,1'-carbonyldiimidazole and epoxy compounds. The use of epoxy compounds is described in Axen et al. *Acta Chem. Scand.*, B29: 471—474 (1975). Epichlorohydrin or 1,4-bis(2,3-epoxypropoxy) butane reacts with a hydroxyl group of an agarose gel to form an epoxide gel. The epoxide gel is then reacted with sodium thiosulfate to give a thiosulfate ester gel, which is then reduced by dithiothreitol to give a modified agarose gel containing a thiol group. This so-called thiol gel is then converted to a 2-pyridyl disulfide gel by means of 2,2'-dipyridyl disulfide. A solution of urease is then passed through a column of the disulfide gel to obtain an enzyme conjugate of high protein content and high catalytic activity. One disadvantage of this procedure is that the epoxy substituted polymer is not stable enough to store.

More recently the use of various organic sulfonates has found wide use in preparing immobilized affinity ligands. For example, Nilsson et al., *Eur. J. Biochem.*, 112: 397—402 (1980) describes the coupling of a number of biomolecules to agarose gels by means of p-toluenesulfonyl chloride. The biomolecules used includes nucleic acids and enzymes.

The use of other organic sulfonyl halides and the use of other hydroxyl group carrying supports are described in Nilsson et al., *Biochem. Biophys. Res. Comm.*, 102: 449—457 (1981). The most active sulfonyl halide appears to be 2,2,2-trifluoroethanesulfonyl chloride (tresyl chloride). Other hydroxyl group carrying supports mentioned in this reference are cellulose, diol-silica, glycophase-glass, and hydroxyethyl methacrylate.

U.S. Patent No. 4,415,665 to Mosbach et al. teaches a method of covalently binding a biologically active substance containing amino, thiol or aromatic hydroxyl groups directly to a polymeric substance containing at least one hydroxyl group by forming a reactive sulfonate derivative of the polymeric substance and then reacting the thus activated polymeric substance directly with the biologically active organic substance. Although the use of sulfonyl halides has proven to be advantageous in many ways, the cost of the more active organic sulfonyl halides tends to be prohibitive and tresyl chloride, being a liquid, is less convenient to handle.

The purification of enzymes using consecutive thiol-disulfide interchange reactions is described in Carlsson et al., *Acta Chem. Scand.*, B30: 180—182 (1976) in a communication in which urease is covalently bonded to agarose-2-pyridyl disulfide. Although this procedure is effective in carrying out the covalent chromatographic purification of urease, the preparation of the agarose-2-pyridyl disulfide involves a combination of steps utilizing an unstable epoxide derivative.

Mukaiyama et al. discloses the use of 1-methyl-2-alkoxypyridinium salts as reagents for preparing various 2-pyridyl sulfides. Mukaiyama et al., *Chem. Lett.*, 1159—1162 (1975).

Hojo et al. successfully demonstrated the conversion of various alcohols to the corresponding thiolalcohol by reacting the alcohol with 1-methyl-2-fluoropyridinium salts and sodium N,N-dimethyldithiocarbamate followed by reductive cleavage. The alcohols exemplified by these authors, including carbohydrates and steroids, were low molecular weight monomeric alcohols. Hojo et al. *Chem. Lett.*, 437—440 (1977).

A convenient method has now been found for preparing covalent chromatographic matrices utilizing a hydroxyl containing polymer which has been activated by reaction with 2-fluoro-1-methylpyridinium toluene-4-sulfonate (FMP). The activated hydroxyl containing polymer can be used in forming covalent bonds with various ligands containing amino and sulfhydryl groups. However, the covalently bound ligands are difficult to remove from the polymeric matrix. Therefore, it was found desirable to bind the ligand to the polymer in a manner such that the ligand could be readily removed when desired. The procedure involves conversion of the activated polymer to a thiol gel, that is, a sulfhydryl group containing polymer. The thiol gel can then be reacted with 2,2′-dipyridyl disulfide to form a 2-pyridyl disulfide derivative of the polymer. Thiol-disulfide interchange with the sulfhydryl group containing ligand causes the ligand to be linked to the polymer by means of a disulfide linkage. Removal of the ligand when desired can be readily accomplished by reduction of the disulfide linkage with a thiol such as dithiothreitol.

Two different routes to the thiol gel are available. In one, sodium dimethyl dithiocarbamate is used to convert the activated polymer to the corresponding dimethyl dithiocarbamyl derivative, which, by means of reductive cleavage is converted to the desired sulfhydryl substituted polymer, hereinafter referred to as the DS-gel.

Another route to a thiol gel involves treatment of the FMP activated polymer with dithiothreitol to form a dithiothreityl gel, a thiol gel, hereinafter referred to as the DTT-gel, in which the free sulfhydryl group is 4 carbon atoms removed from a thioether linkage to the polymer.

Depending upon the particular application for which the covalent chromatographic matrix is intended, either the DS-gel or the DTT-gel may be ideally suited for the particular chromatographic procedure which is to be carried out. For example, the DTT-gel may be particularly adapted for use in those instances where the ligand contains bulky groups which might prevent it from approaching close enough to the polymer to attack the disulfide linkage, were it not for the space provided between the polymer surface and the disulfide linkage by the intervening 4 carbon atom chain.

## Brief description of the drawings

Figure 1 is a schematic flow sheet illustrating the process for preparation of an activated polymer according to the present invention.

Figure 2 is an elution curve illustrating the affinity purification of rabbit antiserum to 2,4-Dinitrophenyl bovine serum albumin using 2, 4-DNP-lysine conjugated Sepharose CL-4B as the affinity matrix.

Figure 3 is an elution curve illustrating the affinity purification of rabbit anti-tobramycin serum using tobramycin conjugated Sepharose CL-4B as the affinity matrix.

Figure 4 is a schematic flow sheet illustrating the various routes to the preparation of a thiol gel.

Figure 5 is an elution curve showing the absorbance of various fractions of eluate during the purification of jack-bean unrease by covalent chromatography.

Figure 6 is a reaction curve showing the course of the immobilization of *E. Coli* beta-galactosidase on 2,2′-dithiodipyridyl activated thiol (DDT) gel and the reversal thereof upon the addition of dithiolthreitol.

## Detailed description

The polymeric carrier can be a water insoluble or water soluble polymeric substance and the choice of the carrier is not critical for carrying out the process of the present invention. In principal, any type of carrier can be used which has a polymeric nature and contains at least one hydroxyl group bonded to a carbon atom which is available for activation and coupling. The carrier is chosen with regard to the requirements in the individual situation, primarily with regard to the type of ligand to be coupled and the intended use of the coupling product. The carrier may be comprised of natural, semi-synthetic or synthetic materials containing hydroxyl groups. Examples of important carrier materials are polysaccharides and polysaccharide containing materials, for example, cellulose agarose, dextran and cross-linked products thereof. Examples of synthetic carriers are polyethylene glycol, polyvinyl alcohol, polyhydroxyethyl methyl acrylate and the like. It is, of course, also possible to use carriers which normally do not contain hydroxyl groups but which, by suitable treatment, can be provided with such groups. An example is silica particles, to the surface of which have been bonded groups containing at least one hydroxyl group bonded to a carbon atom.

The activation of hydroxyl containing polymeric carriers can be carried out in the presence of a slight excess of a tertiary amine such as triethylamine or tributylamine in dry polar organic solvents such as acetonitrile, acetone or tetrahydrofuran. FMP reacts rapidly, usually in about 1—15 minutes at ambient conditions of temperature and pressure, for example, at about 22—35°C, with hydroxyl groups of various polymeric materials to form 2-alkoxy-1-methylpyridinium salts, which react readily with amino or sulfhydryl groups of various nucleophiles suitable for use as affinity ligands. Other 2-halo-1-methylpyridinium salts such as 2-chloro-1-methylpyridinium salts can be used, but the 2-fluoro-1-methylpyridinium salt is preferred because of its greater reactivity.

Unreacted activating agent such as FMP can be easily washed from the polymeric carrier with a dilute acid such as dilute HCl, for example, with 2 mM HCl, to purify and stabilize the activated polymer without causing hydrolysis of the activated hydroxyl groups.

The FMP-activated polymeric carrier was found

to be stable for at least 4 months when stored at 4°C in 2 mM HCl. The activated polymeric carrier can also be stored in dilute mineral acids such as 2 mM phosphoric acid or in dry form, if desired. Activation densities of 4—7 micromoles per ml of gel are routinely obtained.

The coupling method of the present invention is generally applicable to organic ligands containing the indicated amino or sulfhydryl groups. For example, primary amino, secondary amino or sulfhydryl groups may be utilized for the desired coupling to the activated polymeric hydroxyl containing polymer. Likewise, salts of sulfhydryl group containing compounds such as Na salts thereof are useful for this purpose. In general, the product selected for coupling should be a good nucleophile, so that the coupling reaction can be carried out smoothly. Any group capable of displacing the 1-methyl-2-pyridoxy group from the polymeric carrier is satisfactory for use as a ligand. Thus, the ligand may contain any aliphatic, aromatic heterocyclic, or heteroaromatic radical or any radical which is a combination of the foregoing, so long as the resulting ligand will have functional groups available for coupling. Of special interest are biologically active ligands, for example, proteins, such as enzymes; antibodies and antigens; amino acids; thiol compounds; cofactors; nucleotides; polynucleotides; haptens and many other types of biologically active ligands, especially those having biospecific affinity to another substance which can be used, for example, for affinity chromatographic purposes.

The reaction scheme is illustrated in Figure 1 of the drawings wherein the symbol

$$—CH_2—OH$$

represents a polymeric carrier having at least one $—CH_2—OH$ group, $TsO^-$ represents the toluene-4-sulfonate ion, TEA represents triethylamine, $L—NH_2$ represents an amino group containing ligand and L—SH represents a sulfhydryl group containing ligand.

Coupling can be carried out under varying conditions of temperature and pH and can be performed in aqueous reaction media as well as in polar organic solvents. Reaction conditions are not critical for either the activation step or the coupling step and are primarily chosen with regard to the sensitivity of the reactant and to practical considerations of convenience. Mild reaction conditions are preferred. It is, for example, often suitable to work at ambient temperatures and pressures and, in the case of an aqueous reaction medium, the pH is usually close to neutral, for example, pH 8—9. The degree of coupling to the hydroxyl groups of the carrier can be varied as needed by stoichionmetric adjustment to utilize essentially all available hydroxyl groups of whatever part thereof is desired. Coupling efficiencies of 70—80% are realized routinely.

Unreacted activated groups remaining after coupling, which might impede further utilization of the coupled polymer, can be removed by suspending the coupled polymer in 0.2M Tris-HCl, pH 9, at room temperature, for 2 hours. Other nucleophiles such as ethanolamine or mercaptoethanol can also be used for this purpose.

The preferred coupling process of the present invention involving the use of a thiol gel will be described in detail with reference to Figure 4, wherein there are shown the various steps involved in the overall reaction scheme.

The first step in the process shown is the reaction of hydroxyl containing polymer having at least one reactive hydroxyl group (Formula 1) with 2-fluoro-1-methylpyridinium toluene-4-sulfonate (Formula 2), which has been described above. The resulting 2-alkoxy-1-methylpyridinium salt (Formula 3) will be referred to at times as the activated polymer or activated gel. The activated polymer is readily attacked by nucleophiles, since the 1-methyl-2-pyridoxy group is a good leaving group being readily converted to 1-methyl-2-pyridone (Formula 4) upon nucleophilic substitution by a ligand.

One sequence of reactions leading to the production of a thiol gel, wherein the sulfhydryl group is directly bonded to a carbon atom of the polymer, utilizes sodium dimethyl dithiocarbamate (Formula 5) to react by nucleophilic substitution upon the activated polymer to produce a dimethyl dithiocarbamayl derivative of the polymer (Formula 6). Reaction of the activated gel with sodium dimethyl dithiocarbamate occurs readily in an organic solvent such as N,N-dimethyl formamide (DMF). Other solvents which may be used include acetonitrile, acetone and tetrahydrofuran. The reaction is readily conducted under ambient conditions of temperature and pressure in a period of about 12 to 20 hours.

Reduction of the dimethyl dithiocarbamyl derivative of the gel results in a thiol gel having a sulfhydryl group directly bonded to a carbon atom of the gel (Formula 7). Reduction can be readily accomplished using any standard reducing agent such as sodium borohydride, lithium aluminum hydride or sodium dithionite. The reaction takes place readily under ambient conditions of temperature and pressure and in a period of time of about 6 hours to 12 hours. The resulting DS-gel has a sulfhydryl group content of 5—15 micromole per gram of dry gel.

Another route to a thiol gel involves the reaction of the activated gel (Formula 3) with dithiothreitol (Formula 8) to produce the DTT-gel, (Formula 9) a thiol gel having a sulfhydryl group, which is separated by a 4 carbon atom chain from a thioether linkage to the polymer. The activated gel and dithiothreitol are readily reacted by mixing in the presence of a base such as sodium bicarbonate or a tertiaryamine, such as triethylamine or tributylamine. The reaction is conducted under ambient conditions of temperature and pressure and is complete in a period of about 4 to 8 hours.

The resulting DTT-gel, like the DS-gel, can then be used as a covalent chromatographic matrix. However, before proceeding with the use thereof,

it is important that any unreacted 1-methylpyridoxy activating groups be removed from the DS-gel or DTT-gel in order to control the desired course of the reaction with the ligand which is the subject of the covalent chromatography. Removal of unreacted activating groups is readily accomplished by using a reactive ligand such as Tris-HCL, for example, 0.2 M Tris-HCl, pH 9, ethanolamine, mercaptoethanol or other suitable reactive ligand which will not, however, react with the sulfhydryl group of the thiol gel.

The thiol gel is activated in order to conduct the thiol-disulfide interchange which is responsible for the binding of the desired ligand to the gel. Activation is most readily achieved by reacting the thiol gel with 2,2'-pyridyl disulfide (Formula 10). The reaction proceeds under ambient conditions of temperature and pressure and is complete in a period of about 1 to 3 hours.

The activated DS-gel (Formula 11) or the activated DTT-gel (Formula 12) is then reacted with the desired ligand, represented in Figure 4 as an enzyme having a free sulfhydryl group (Formula 13) in order to form a disulfide linkage between the gel and the enzyme. In the case of the DS-gel, the disulfide linkage binds the enzyme directly to a carbon atom of the gel (Formula 14), whereas in the case of the DTT-gel, the disulfide linkage binds the enzyme to a carbon atom chain linked by a sulfur atom to a carbon atom of the gel (Formula 15). In both cases, 2-thiopyridone (Formula 16) is displaced from the activated thiol gel. Formulae 14 and 15 illustrate immobilized enzymes. It should be appreciated that many other ligands can be similarly immobilized in accordance with the above described process. Reaction of the ligand with the activated thiol gel is readily carried out under ambient conditions of temperature and pressure with suitably pufified and buffered ligand. The reaction is complete in about 2 to 6 hours.

The immobilized enzyme will release the enzyme (Formula 13) upon treatment with a reducing agent (Formula 17) such as cysteine, dithiothreitol or mercaptoethanol. Release of the enzyme occurs readily at ambient conditions of temperature and pressure.

The invention will be better understood with reference to the following examples which are intended for purposes of illustration and are not to be construed as in any way limiting the scope of the present invention, which is defined in the claims appended hereto.

Example 1

Activation of cross-linked agarose with 2-fluoro-1-methylpyridinium toluene-4-sulfonate (FMP).

Sepharose CL-4B was washed successively with 20 gel volumes of distilled water and mixtures of acetone and water having volume-to-volume ratios of 25:75, 50:50, 72:25 and 100% acetone and finally, with 10 gel volumes of dry acetone. A quantity of 50 g of the washed Sepharose gel was suspended in 50 ml of dry acetonitrile mixed with 1 ml of dry triethylamine and stirred vigorously at room temperature. A solution of 3 g of FMP in 40 ml of dry acetonitrile and 1.5 ml of dry triethylamine was added to the gel suspension in 5 ml portions. After 10 minutes the gel was washed with 10 gel volumes of mixtures of acetone and 2 mM HCl having a volume-to-volume ratio of 75:25, 50:50, 25:75 and undiluted 2 mM HCl.

Example 2

Coupling of N,ε-2,4-Dinitrophenyl-L-lysine to activated gel.

A quantity of 100 mg of N,ε-2,4-Dinitrophenyl-L-lysine was dissolved in 30 ml of 0.2 M $NaHCO_3$. To this solution was added 5 g of the FMP-activated gel prepared according to the method of Example 1. The resulting suspension was stirred at room temperature for 15 hours. The gel was then removed from the suspension and washed with 500 ml of 0.2 M $NaHCO_3$, resuspended in 100 ml of 0.1 M Tris-HCl, pH 8, and stirred at room temperature for 2 hours. The gel was then washed with 500 ml of 1 M NaCl and 500 ml of 0.1 M sodium phosphate buffer, pH 7.5, containing 0.15 M NaCl (PBS).

Example 3

Coupling to tobramycin to activated gel

The FMP-activated gel prepared according to Example 1 and stored in 2 mM phosphoric acid solution was removed from the phosphoric acid and a 20 ml quantity of the FMP-activated gel was added to 10 ml of 0.5 M $NaHCO_3$ containing 0.2 millimole to tobramycin.

The gel suspension was gently stirred at room temperature for 24 hours and then washed with 500 ml phosphate buffered saline (PBS). The washed gel was suspended in 0.1 M Tris for 15 minutes to deactivate any unreacted activated hydroxyl groups. Then the gel was washed with 500 ml of PBS, 1000 ml PBS containing 1 M NaCl, and finally with 500 ml of PBS.

In order to determine the extent of activation of the hydroxyl groups of the polymeric carrier, the density of activated expressed in units of micromoles per ml of gel is measured. Since 1-methyl-2-pyridone is released from the activated gel upon coupling with nucleophiles, it is possible to quantitatively determine the density of activation by means of the absorbance at 297 nm of the solution in which the coupling reaction is conducted. At this wavelength in 0.2 M Tris-HCl, pH 9, 1-methyl-2-pyridone has a molar extinction of 5900. The density of activation can then be determined by suspending 1 ml of activated gel in 2 ml of 0.2 M Tris-HCl, pH 9, and stirring gently at room temperature for 10 hours. Upon centrifuging the resulting gel suspension the absorbance of the supernatant at 297 nm is measured and compared with the absorption of solutions of 1-methyl-2-pyridone of known concentration. Using the activation procedure herein described, an activated gel with activation density of 40—70 micro-moles/ml of Sepharose CL-4B was obtained.

The coupled products prepared according to the present invention are useful in various applications where it is desired to have a ligand, such as a biologically active material, immobilized by attachment to a polymeric carrier, for example, in affinity purification, covalent chromatography, and reversible and irreversible covalent immobilization of biomolecules. The use of the coupling products in affinity chromatography is illustrated in the following examples.

Example 4

Purification of rabbit anti-serum to 2,4-Dinitrophenyl bovine serum alblumin using N,ε-2,4-Dinitrophenyl-L-lysine conjugated Sepharose CL-4B as the affinity matrix.

A quantity of 2 ml of rabbit anti-DNP serum was centrifuged to remove particulates. The supernatant was applied onto a 0.5×20 cm column of N,ε-2,4-Dinitrophenyl-L-lysine conjugated Sepharose CL-4B prepared according to Example 2. After applying the anti-serum, the column was washed extensively with PBS until the absorbance at 280 nm of the eluate was less than 0.02. Then 0.1M glycine-HCl, pH 2.5, containing 10% by volume of tetrahydrofuran was used as the eluant. The antibody appeared in the third 5 ml. fraction after applying glycine-HCl buffer. The results of this experiment are shown in Figure 2.

Example 5

Affinity purification of rabbit anti-to-bramycin serum using tobramycin conjugated Sepharose CL-4B as the affinity matrix.

A quantity of 1m of rabbit anti-tobramycin serum was diluted tenfold with PBS. The diluted antiserum was centrifuged at 2000 rpm for 30 minutes to remove solid debris. The entire supernatant was applied to a 0.5×20 cm column of Sepharose CL-4B conjugated with tobramycin prepared as described in Example 3. The column was washed with PBS until the absorbance of the eluate at 280 nm was less than 0.02. The antibodies were eluted with 0.1 M glycine-HCl, pH 2.5 containing 10% tetrahydrofuran in 7 ml. fractions. The results of this experiment are shown in Figure 3.

As shown in Examples 4 and 5 above, the ligand coupled polymeric carries prepared according to the process of this invention are useful as affinity absorbants for purifying various materials which are capable of forming affinity bonds with the ligand coupled polymer. For example, the ligand coupled polymers can be used for purification of antibodies in which case elution of the antibodies absorbed upon the affinity matrix can be accomplished without causing leakage of the ligand from the matrix. The ligand coupled matrix is also characterized by stability during storage. For example, the N,ε-2,4-Dinitrophenyl-L-lysine coupled Sepharose CL-4B was stored in phosphate buffered saline at 4°C without losing any ligand by leakage from the ligand-coupled matrix.

As mentioned above, an important advantage of the coupling method of the present invention is that the coupled substance, that is, the ligand, is covalently bonded directly to a carbon atom of the polymeric carrier, which makes splitting off by hydrolysis unlikely. Furthermore, no additional charge is introduced during the coupling reaction as is the case in some of the prior art coupling methods. Cross-linking of the carrier material, which is a common and undesired side effect of coupling methods previously available, is avoided by means of the coupling method of the present invention.

Example 6

Preparation of thiol gel-dimethyl dithiocarbamate method (DS-gel)

A 1 g sample of the dry activated gel of Example 1 was washed with 100 ml dry N,N-dimethylformamide (DMF) and added to 100 ml DMF containing 1.8 g sodium dimethyl dithiocarbamate. The gel suspension was stirred at room temperature for 16 hours, then washed with 100 ml dry DMF and resuspended in 50 ml dry DMF. Sodium borohydride was added to the suspension in the amount of 380 mg. The gel was continuously stirred at room temperature for 4 hours. Then another 380 mg sodium borohydride was added. The suspension was stirred at room temperature for 4 more hours and then the gel was washed with 200 ml DMF, 1000 ml 2 mM HCl, 500 ml 0.5 N NaCl and 500 ml 2 mM HCl. The sulfhydryl content of the thiol gel was determined by means of 5,5′-dithiobis (2-nitrobenzoic acid) as described in G.L. Ellman, *Arch. Biochem, Biophys*, 82:70—77 (1959) and determined to be 9 micromole/g of dry gel.

Example 7

Preparation of thiol gel-dithiothreitol method (DTT-gel)

The dry activated gel of Example 1 in a quantity of 1 gram was added to a stirred solution of 1 M dithiothreitol (DTT) in 0.2 M $NaHCO_3$. The suspension was stirred at room temperature for 5 hours. The gel was then washed with 500 ml 0.2 M $NaHCO_3$, 500 ml distilled water and 1000 ml 2 mM HCl. The sulfhydryl content of the gel was found to be 6 micromole/g of dry gel.

Example 8

Preparation of activated thiol gel.

The DS-gel of Example 6 or DTT-gel of Example 7 was washed with 60% acetone-40% 0.05 M $NaHCO_3$ 1 mM in ethylenediaminetetraacetic acid (EDTA). The washed gel was then reacted with 0.3 M 2,2′-dipyridyl disulfide.

Example 9

Covalent chromatographic purification of urease

Partially purified jack-bean urease in the amount of 1 g was added to 40 ml 0.1 M Tris-HCl, pH 7.4 containing 1 mM EDTA and 5 mM dithiothreitol (DTT). The resulting suspension was stirred at 4°C for 1 hour and then centrifuged to remove the particulate fraction. A volume of 5 ml

of the cloudy supernatant was passed through a Sephadex G-50C 1×50 cm column which was equilibrated with 0.05 M Tris-HCl, pH 7.4 containing 0.5 mM EDTA. This procedure insured that any dithiothreitol present would be removed in order to prevent interference during the subsequent covalent chromatographic procedure.

A 5 ml volume of the DTT-free eluate was applied to a 0.5×20 cm column of the activated DS-gel of Example 8. The column was then washed with 0.05 M Tris-HCl, pH 7.4, containing 0.5 mM EDTA until the absorbance at 280 nm of the eluate was less than 0.1 and with 0.05 M Tris-HCl, pH 7.4 containing 0.05 mM EDTA and 0.05 M NaCl until the absorbance was less than 0.02. The enzyme urease was eluted from the column by 0.05 mM Tris-HCl, pH 7.4, containing 0.05 mM EDTA and 20 mM dithiothreitol (DTT). Urease activity was assayed by measuring the rate of disappearance of NADH absorbance at 340 nm through a glutamic dehydrogenase coupled reaction. The substrate solution used in the reaction contained 0.05 M Tris-HCl, pH 7.4; 0.5 mM EDTA; 1 mM ADP; 1 mM alpha-ketoglutarate; 50 mM urea and 50U glutamic dehydrogenase. The assay was initiated by adding 5—10 microliters of enzyme solution to 2 ml of substrate solution. The results of the immobilization of the urease upon the column and the elution from the column are shown in Figure 5.

From the solid curve of Figure 5 it can be seen that most of the UV absorbing materials pass through the column unretarded. These materials were eluted in the first 11 fractions. Upon application of buffer containing a high concentration of NaCl, a small amount of additional UV absorbing material was eluted in fractions 15—20. The dotted line curve representing the urease activity shows that no urease activity was detected until DTT was applied to the column. After DTT was applied, starting at fraction 24, strong UV absorbing material was eluted in fractions 29—33, as is shown in the curve. The application of DTT released not only the enzyme but also 2-thiopyridone, which is also strongly absorbing at 280 nm. That the UV absorbing material constituted mostly 2-thiopyridone (approximately 90%) was established by dialysis of these fractions against 2000 ml 0.05 mM EDTA in 0.05 M Tris-HCl, pH 7.4, which resulted in an average 34-fold reduction in their UV absorbance. Urease activity also increased at the same time as the increase in UV absorbance. A single passage of the enzyme solution through the activated thiol gel resulted in an 11-fold purification with 83% recovery of the total enzyme. The purified enzyme was found to have a specific activity of 770 units/mg.

Similar results can be obtained using the activated DTT-gel of Example 8.

The following example illustrates the reversible immobilization of a biologically active material by means of covalent linkage through a disulfide bridge to the activated thiol gel of the present invention. It should be pointed out that immobilization of a biologically active material for the purpose of providing such material in a form which is convenient to use requires that the disulfide bridge be formed utilizing a sulfhydryl group which is not essential to the activity of the biologically active material.

Example 10

Reversible immobilization of beta-galactosidase

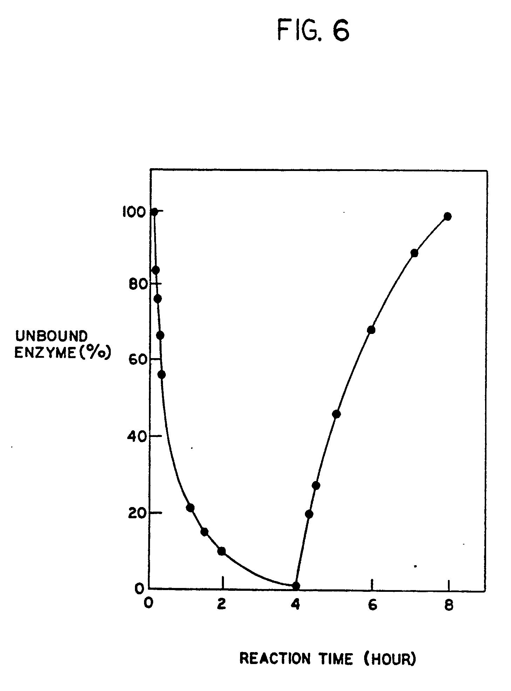

*E. Coli* beta-galactosidase in a quantity of 5 mg was dissolved in 10 ml of 0.1 M sodium phosphate buffer, 0.15 M in NaCl, pH 7.4. Wet activated DTT-gel prepared according to the procedure of Example 8 quantity of 2.5 g was added to the enzyme solution. The resulting suspension was stirred at room temperature. At timed intervals, 0.1 ml of the gel suspension was withdrawn and centrifuged at 2500 rpm for 1 minute. The supernatant was diluted 50× with phosphate buffer and 0.25 ml of the diluted supernatant was assayed for beta-galactosidase in 2 ml o-nitrophenyl-beta-D-galactopyranoside solution. After 4 hours incubation at room temperature total immobilization of the beta-galactosidase with full activity was realized. At that time 20 mM DTT in 0.05 M phosphate, pH 7.7, was mixed with the immobilized enzyme and samples were withdrawn and assayed for beta-galactosidase. As shown in Figure 6, the activity of beta-galactosidase increased until at 8 hours reaction time a total recovery of enzyme was realized.

The foregoing description of the invention has been directed to particular preferred embodiments for purposes of explanation and illustration. It will be apparent, however, to those skilled in the art that many modifications and changes in the methods and materials may be made without departure from the scope and spirit of the invention. For example, other hydroxyl containing polymeric carriers and other ligands may be used. Particular affinity systems described herein have been chosen for convenience and are not intended to be limiting. It is the applicant's intention in the following claims to cover all such equivalent modifications and variations as fall within the true scope and spirit of the invention.

**Claims**

1. A process for activating a hydroxyl group of a polymeric substance containing at least one hydroxyl group comprising:

reacting a polymeric substance containing at least one hydroxyl group with 2-fluoro-1-methylpyridinium toluene-4-sulfonate, and

recovering a polymeric product wherein at least some of the hydroxyl groups of the polymer have been converted to 1-methyl-2-pyridoxy groups.

2. A process of covalently binding:

(A) an organic ligand that contains at least one substituent selected from the group consisting of primary amino groups, secondary amino groups and sulfhydryl groups;

directly to a:

(B) polymeric substance containing at least one hydroxyl group; comprising the steps of:

(1) first forming a reactive derivative by reacting

(i) 2-fluoro-1-methylpyridinium toluene 4-sulfonate with

(ii) a polymeric substance containing at least one hydroxyl group, wherein at least one reacting hydroxyl group is bonded to carbon atoms in the polymeric substance and then

(2) reacting said reactive derivative directly with the organic ligand as set forth in (A).

**Patentansprüche**

1. Verfahren zur Aktivierung einer Hydroxylgruppe einer polymeren Substanz, die wenigstens eine Hydroxylgruppe aufweist, umfassend:

Einwirkenlassen von 2-Fluor-1-Methylpyridintoluol-4-Sulfonat auf eine polymere Substanz, die wenigstens eine Hydroxylguppe enthält, und

Rückgewinnen eines polymeren Produktes, in welchem wenigstens einige der Hydroxylgruppen des Polymers in 1-Methyl-2-Pryridoxy-Gruppen umgewandelt worden sind.

2. Verfahren zur kovalenten Bindung:

(A) eines organischen Liganden, der wenigstens einen Substituenten enthält, der aus der Gruppe ausgewählt worden ist, die primäre Aminogruppen, sekundäre Aminogruppen und Sulfhydrylgruppen umfaßt;

direkt mit

(B) einer polymeren Substanz, die wenigstens eine Hydroxylgruppe enthält; aufweisend die Stufen:

(1) zuerst Umformen eines reaktiven Derivates durch Umsetzen

(i) von 2-Fluor-1-Methylpyridintoluol-4-Sulfonat

ii) mit einer polymeren Substantz, die wenigstens eine Hydroxylgruppe aufweist, worin wenigstens eine reagierende Hydroxylgruppe an Kohlenstoffatome in der polymeren Substanz gebunden ist, und dann

(2) Umsetzen des reaktiven Derivats direkt mit dem organischen Liganden wie in der Stufe (A).

**Revendications**

1. Procédé d'activation d'un groupe hydroxyle d'une substance polymère comportant au moins un groupe hydroxyle qui consiste:

à faire réagir une substance polymère comportant au moins un groupe hydroxyle sur du toluène-4-sulfonate de 2-fluoro-1-méthylpyridinium, et

à recueillir un produit polymère, au moins certains des groupes hydroxyle du polymère ayant été transformés en groupe 1-méthyl-2-pyridoxy.

2. Procédé de liaison par covalence de:

(A) un ligand organique qui comporte au moins un substituant choisi parmi les groupes amine primaire, amine secondaire et les groupes sulfhydryle;

directement à une:

(B) substance polymère qui comporte au moins un groupe hydroxyle; caractérisé en ce qu'il consiste

(1) à former d'abord un dérivé réactif par réaction de:

(i) toluène-4-sulfonate de 2-fluoro-1-méthylpyridinium sur

(ii) une substance polymère comportant au moins un groupe hydroxyle, au moins un groupe hydroxyle réactif étant relié à des atomes de carbone de la substance polymère, puis

(2) à faire réagir ce dérivé réactif directement sur le ligand organique tel qu'indiqué en (A).

FIG. 1

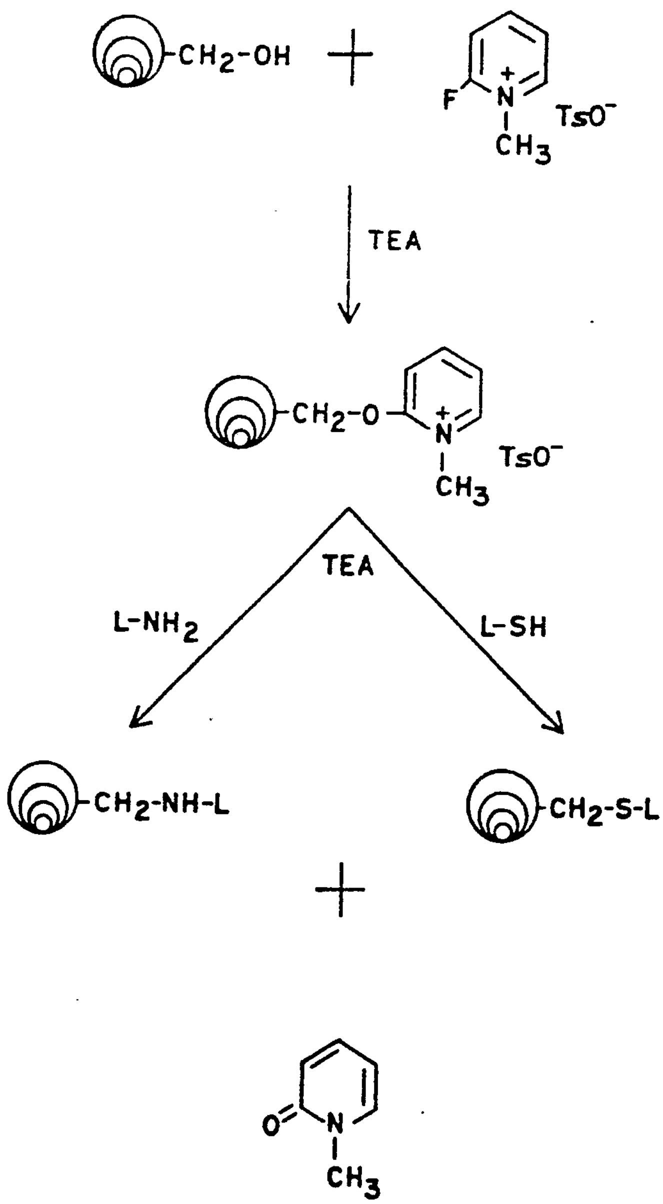

FIG. 2

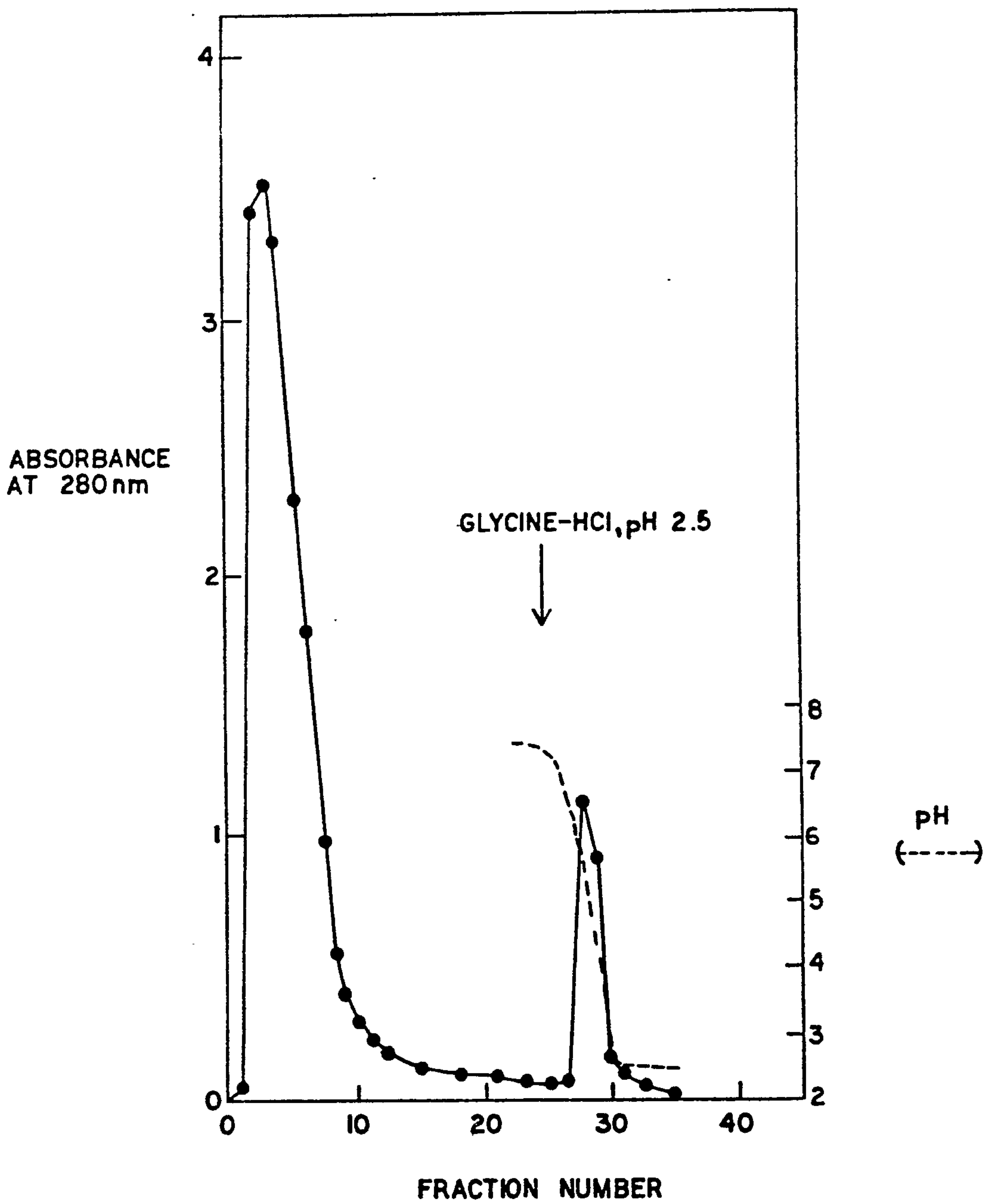
ABSORBANCE AT 280nm
GLYCINE-HCl, pH 2.5
pH (------)
FRACTION NUMBER
0
1
2
3
4
10
20
30
40
5
6
7
8

FIG. 3

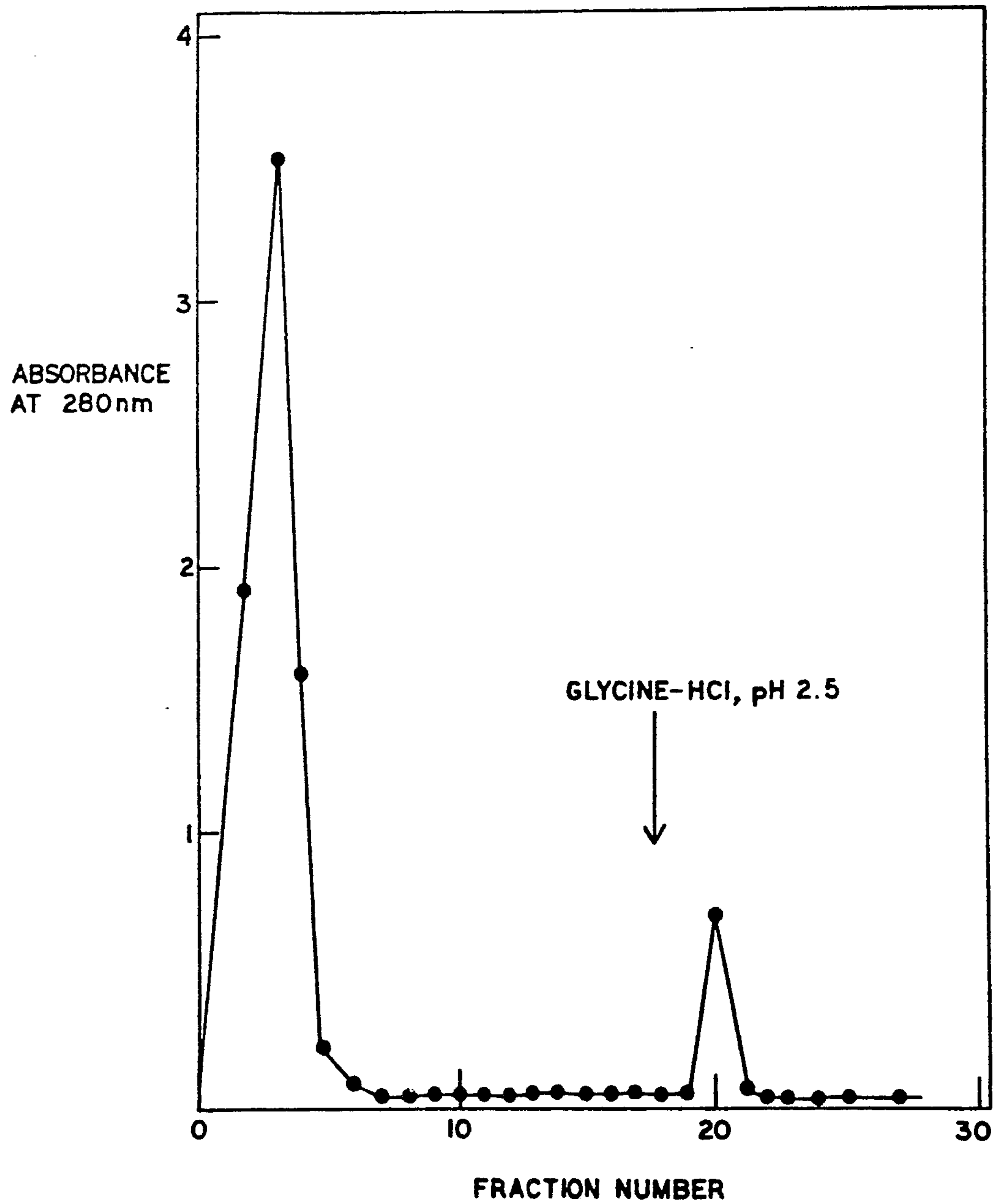
4
3
2
1
ABSORBANCE AT 280nm
GLYCINE-HCl, pH 2.5
0
10
20
30
FRACTION NUMBER

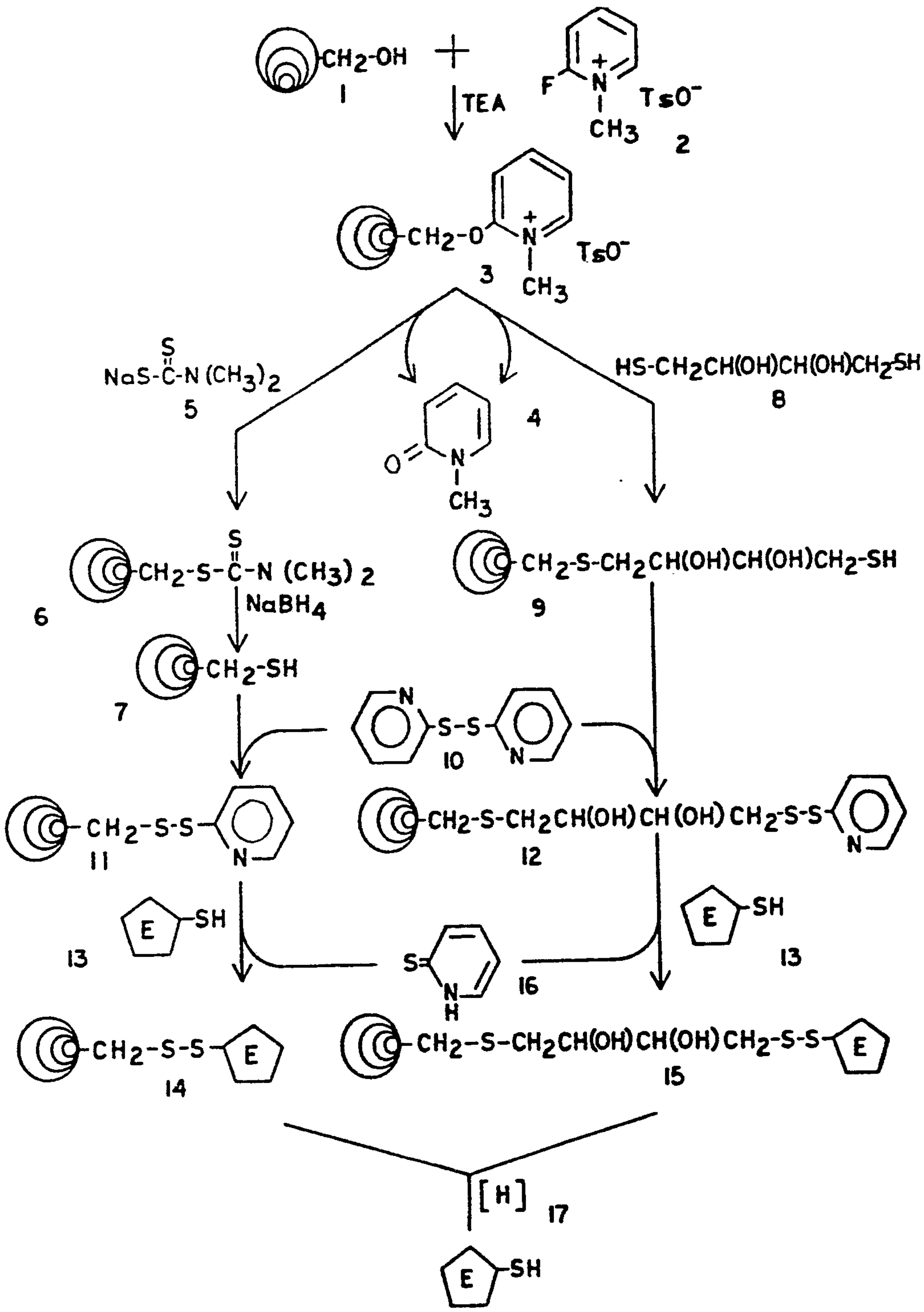


13

FIG. 4

FIG. 5

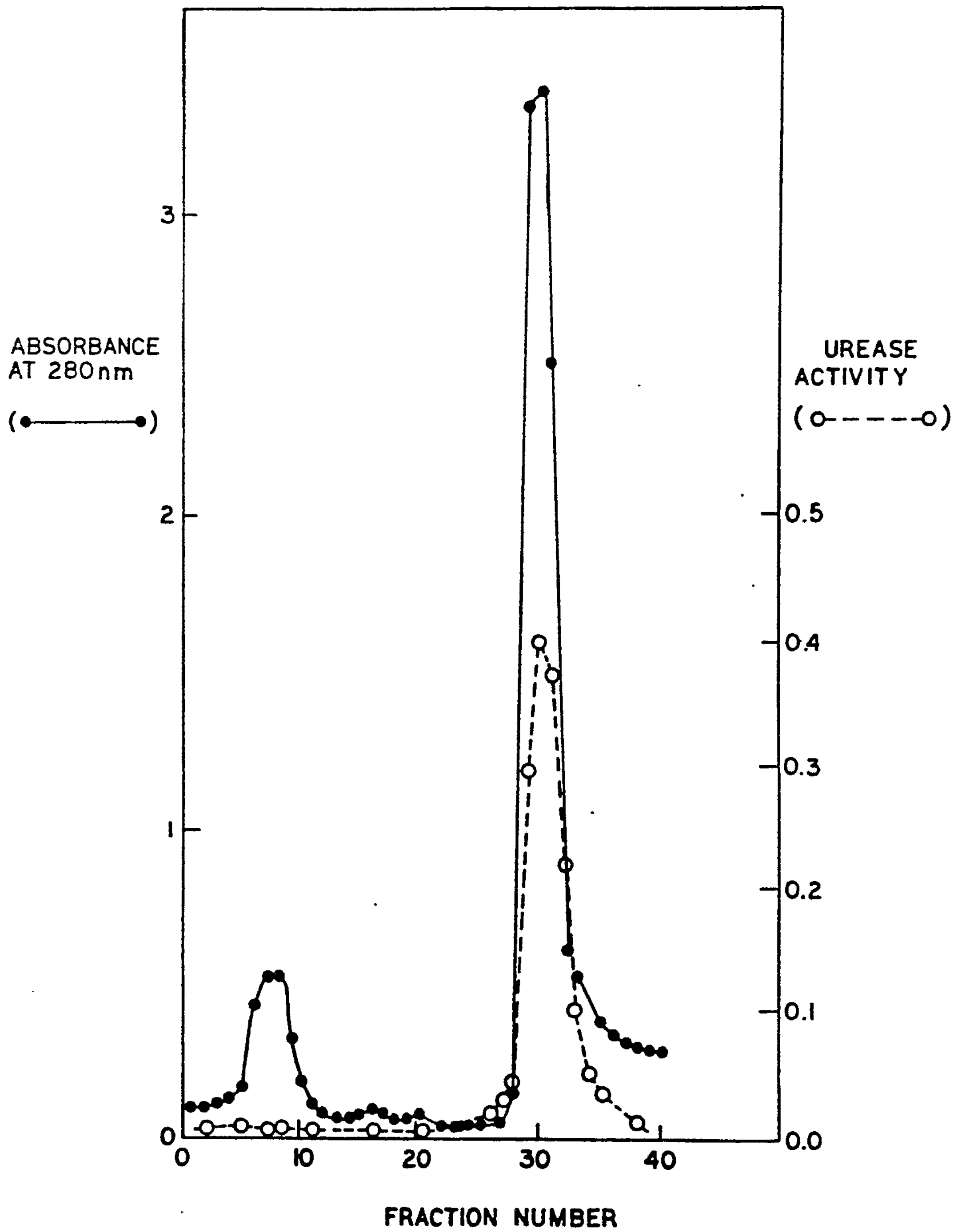
ABSORBANCE
AT 280nm
UREASE
ACTIVITY
3
2
1
0
0.5
0.4
0.3
0.2
0.1
0.0
0
10
20
30
40
FRACTION NUMBER

FIG. 6